**Europäisches Patentamt**

**European Patent Office**

**Office européen des brevets**

(11) Publication number: **0 413 025 A1**

# EUROPEAN PATENT APPLICATION
## published in accordance with Art. 158(3) EPC

㉑ Application number: **89906457.0**

㉒ Date of filing: **03.06.89**

㊻ International application number:
**PCT/JP89/00564**

㊺ International publication number:
**WO 89/11828 (14.12.89 89/29)**

㉛ Int. Cl.⁵: **A61B 17/36**

㉚ Priority: **04.06.88 JP 138142/88**
**06.06.88 JP 138760/88**

㊸ Date of publication of application:
**20.02.91 Bulletin 91/08**

㊷ Designated Contracting States:
**DE FR GB IT NL**

⑦ Applicant: **SUMITOMO ELECTRIC INDUSTRIES, LTD**
**5-33, Kitahama 4-chome Chuo-ku**
**Osaka 541(JP)**

(72) Inventor: **SOGAWA, Ichiro Osaka Works of Sumitomo Electr.**
**Ind., Ltd. 1-3, Shimaya 1-chome Konohana-ku Osaka-shi, Osaka 554(JP)**
Inventor: **NIWA, Shin-ichiro Osaka Works of Sumitomo Electr.**
**Ind., Ltd. 1-3, Shimaya 1-chome Konohana-ku Osaka-shi, Osaka 554(JP)**
Inventor: **YOTSUYA, Koro Osaka Works of Sumitomo Electr.**
**Ind., Ltd. 1-3, Shimaya 1-chome Konohana-ku Osaka-shi, Osaka 554(JP)**
Inventor: **UEMIYA, Takafumi Osaka Works of Sumitomo Electr.**
**Ind., Ltd. 1-3, Shimaya 1-chome Konohana-ku Osaka-shi, Osaka 554(JP)**
Inventor: **KANAZAWA, Shin-ichi Osaka Works of Sumitomo Electr**
**Ind., Ltd. 1-3, Shimaya 1-chome Konohana-ku, Osaka-shi, Osaka 554(JP)**

㊴ Representative: **Lehn, Werner, Dipl.-Ing. et al**
**Hoffmann, Eitle & Partner Patentanwälte**
**Arabellastrasse 4**
**D-8000 München 81(DE)**

(54) **LASER-AIDED INTRAVASCULAR OPERATION EQUIPMENT.**

㊼ Laser-aided operation equipment for carrying out operation in the blood vessel using laser. In an intravascular laser-aided operation equipment, the interior of the blood vessel is displayed by an image device based on the output light from a catheter (1) that is inserted in the body, and the diseased part is irradiated with a laser beam from a laser device (2) to effect therapy. The catheter (1) is equipped with both an image transmission path (11) for transmitting the image in the blood vessel and a laser guide path (14) for guiding the laser beam to the diseased part in the blood vessel, and is further equipped with a drive controller (7) that controls the whole system. The laser device (2) is suitably driven based on the endoscopic image and the result of spectral analysis to emit the laser beam. Therefore, the therapy is carried out while making sure the diagnostic condition at all times without the need of replacing the fibers.

*Fig. 1*

## LASER OPERATING DEVICE FOR INTRAVASCULAR SURGERY

### FIELD OF THE INVENTION

The present invention relates to a laser operating device for intravascular surgery, and more in particular to a laser operating device for intravascular surgery which is used for removing a diseased part inside a blood vessel using laser lights.

### PRIOR ART

In the prior art, there have been contrived various kinds of medical treatment diagnosis devices and methods thereof for removing e.g a stenosis or occlusion of a blood vessel and an atheroma due to such as an arteriosclerosis.

A bypass operation is the most assured treatment method in which a lesion is completely removed by replacing a blood vessel which includes a diseased part with one of patient's own blood vessels or with an artificial blood vessel for example. However, since this method is followed by a surgery in which vital organism is cut open, the vital body is subjected to a burden and a large amount of costs is needed for the treatment. Moreover, although a drug treatment is also adopted, it is effective only for solution of a thrombus and it has been difficult to remove a stenosis and occlusion of a blood vessel.

Therefore, there has been recently adopted a treatment that a catheter is inserted into a blood vessel from the outside of a body and reached a diseased part so as to directly remove a cause of an obstacle.

One is a treatment that a balloon catheter having a balloon attached on its distal tip portion is used and the balloon is expanded when it is reached the diseased part so that the stenosis of the blood vessel is mechanically expanded. However, since the stenosis is simply expanded, e.g. the diseased focus of the arteriosclerosis or thrombus which causes a stenosis can not be removed and a probability of a relapse of a disease in a short period is high. Moreover, in the cases that the balloon can not be inserted to a blood vessel because the blood vessel is entirely occluded and that the arteriosclerosis is so advanced as to cause a calcification, it becomes difficult to treat with a balloon.

The other is a method using laser light such as YAG laser or argon laser, wherein a metallic or ceramic chip attached to the tip of the catheter is heated by the laser light radiated from the tip of an optical fiber so that the heated chip is pressed onto the diseased part so as to burn out the diseased part. According to this method, though the diseased part can be removed, the control of the light heating power is difficult and if the chip is overheated, a normal vessel wall is damaged or carbonized so that there may cause a new risk of vascular perforation or a new restenosis. Moreover, in case the vessel is tortuous or completely occluded, it is not available because the chip can not be inserted.

Therefore, it is also adopted that the laser light from such as YAG laser, argon laser and excimer laser is directly projected to the diseased part from the tip of the fiber so as to vaporize the diseased part. Since the portion having the laser light projected thereon is directly vaporized, the laser light is available also for a completely occluded diseased part and upon controlling the output of the laser light source, the pulse width and pulse intervals of the pulse laser, it is possible to control the power with high accuracy.

By the way, in the case of diagnosis and treatment of a diseased part in a vessel by means of the intravascular laser operating device of a direct radiation type mentioned above, the treatment is performed in the processes (1) to (6) as follows.

That is to say, (1) image transmission fiber and illumination light transmission fiber are inserted up to the diseased part. (2) Flush liquid including drug liquid, disinfectants and anesthetics, which has little loss in the range of the wave length of the illumination light, is charged into the vessel and is replaced for blood so as to make the vision transparent. (3) The condition in the vessel is displayed on a screen under the condition that the diseased portion is being made transparent. (4) Subsequently, the image transmission fiber is pulled out of the catheter and is shifted into the laser light projecting fiber. (5) The laser light is projected to the diseased part through the laser light projecting fiber so as to remove such as a thrombus. Moreover, at this time, there is also a case that, if necessary, flush liquid including drug liquid, disinfectants and anesthetics, which has little loss in the range of the wave length of the laser light, is charged into the vessel and is replaced for blood so as to make the projection area transparent. (6) The waste produced by projecting the laser light is sucked.

However, in such a method, since it is impossible to observe a diseased part when the laser light is projected, it has been difficult to confirm whether or not the laser light is precisely projected onto the diseased part. Moreover, since, only by an endoscopic method, a formal diagnosis of a diseased part can be merely performed and the judgment of the nature of the condition is difficult, it has been impossible to diagnose with good accuracy. Therefore, it has been difficult to secure a rapidity, safety and soundness of the treatment.

Moreover, since the operator performs the processes 2 to 6 individually after performing the process 1 mentioned above, the operations are troublesome and each device must be manually operated, resulting in waste of excessive nerves.

In addition, it is difficult to control the period and timing of the projection of the laser, and if the projection period and projection timing are so missed as to project too much energy, there is a fear that an inner surface of a vessel besides a diseased portion is injured. If the projection energy is too small, the diseased part remains and the above mentioned operation must be repeated.

Furthermore, in the conventional catheter, since it is necessary to replace the image transmission fiber with the laser light leading fiber, there was a problem that there occurred a difficulty in performing a rapid operation.

The present invention has been made considering the problems mentioned above and has its object to provide an intravascular laser operating device capable of treatment with rapidity, safety and soundness.

SUMMARY OF THE INVENTION

In order to accomplish the object mentioned above, the present invention is an intravascular laser operating device in which the inside of a vessel is displayed on an image device based on the output light of a catheter inserted into a body so that laser light is projected onto a diseased part by a laser device so as to treat the diseased part, comprising;

a catheter accommodating all or one or more parts of (1) to (6), i.e., (1) an illumination light transmission path for transmitting the illumination light into a vessel, (2) an image transmission path for transmitting an image into the vessel, (3) an excitation light transmission path for transmitting excitation light into the vessel, (4) a fluorescent light transmission path for transmitting fluorescent light into the vessel, (5) a laser light leading path for leading laser light to a diseased part in the vessel, and (6) a drug liquid passage path for blowing out transparent drug liquid around the diseased part,

an image device for performing a formal display of an endoscopic image based on the output light obtained through the image transmission path and for performing a spectrum analysis of the fluorescent light obtained through the fluorescent light transmission path so as to display the analyzed result,

an excitation light source for generating excitation light to be entered in said excitation light transmission path,

an illumination light source for generating an illumination light to be entered in said illumination light transmission path,

a laser oscillating unit for generating laser light to be entered in said laser light leading path,

a drug liquid blowout device for charging transparent drug liquid into the drug liquid passage path, and

a drive controller for setting the laser projecting time, drug liquid blowout time and suction time individually and setting the turns and timings of the laser light projection and drug liquid blowout and suction.

However, said drive controller unit may be so constituted as to set the times of the endoscopic display and fluorescent light analysis by the image device and to set the turns and timings of the endoscopic display and fluorescent analysis.

Moreover, since the catheter must be made up to be a thin form with its outer diameter of less than 1.5 mm, it is desired that the components should be made common in usages if possible, therefore, some of the components may be made available in common, for example, one transmission path is used both as the image transmission path and as the fluorescent light transmission path, or the laser light leading path is also used as the excitation light transmission path, furthermore, the excitation light source is also used as the laser oscillating unit.

Moreover, since there is also a case that waste is produced by the laser projection under some condition of the laser projection, there may be provided a suction device for sucking and removing the waste from said drug liquid leading path. And the catheter may comprise a suction path for sucking the waste independently from the drug liquid leading path.

According to the intravascular laser operating device constituted as described above, the state and nature condition of the diseased part in the vessel can be seen through the image diagnosis device and spectrum diagnosis device under the condition that the catheter is inserted in the vessel. And according to these diagnosis results, the conditions of such as the output of the laser light and pulse intervals can be set to be optimum values. And in the case of performing a treatment by laser light, it is possible to proceed to the treatment with the laser light by operating the laser device under the same condition as it is after the endoscopy. In this case, it is also possible to continue the endoscopy or spectrum measurement at the same time of the laser treatment.

In the manner as described above, it becomes possible that the diagnosis and treatment are performed at the same time or in parallel.

Moreover, if the time periods of the laser projection and the drug liquid blowout and suction are individually set and besides the turns and the timings of the drug liquid blowout and suction are set, the processes from the blowout of the drug liquid to the suction of the waste can be automatically performed after the catheter is inserted to a diseased part in a vessel. That is to say, the entire operation time is allocated to a laser projection time, drug liquid blowout time and suction time, subsequently, the respective devices are driven in the turns and timings set by the driving controller, whereby the operation of the operator can be reduced. Moreover, since the timing and time of the laser projection after the blowout of the drug liquid are set, there may be no fear that a healthy vessel is injured or that the diseased part remains. Furthermore, the whole parts of the illumination light transmission path, image transmission path, laser light leading path, drug liquid leading path and suction path are all accommodated in the catheter, whereby there is no need to replace the image transmission path with the laser light leading path, so that the diagnosis and the operation can be rapidly performed. However, to a thin vessel, a thin catheter combined with a part or a plurality of components (1) to (6) mentioned above if necessary may be used to perform the diagnosis and treatment.

Moreover, if the times of the endoscopic display and fluorescent analysis through the image device are set by said driving controller unit and besides the turns and timings of the endoscopic display and fluorescent analysis are set, since the image display is performed in a preset timing, there is no need for operating the image device.

BRIEF DESCRIPTION OF THE DRAWINGS

Fig. 1 is a schematic diagram showing an embodiment of an intravascular laser operating device according to the present invention,

Fig. 2 is a sectional view of a catheter (1),

Fig. 3 is a timing chart of a driving controller (7), and

Fig. 4 is a flow chart of the driving controller.

OPTIMUM EMBODIMENT OF THE INVENTION

An embodiment of the present invention is explained hereinafter with reference to the drawings.

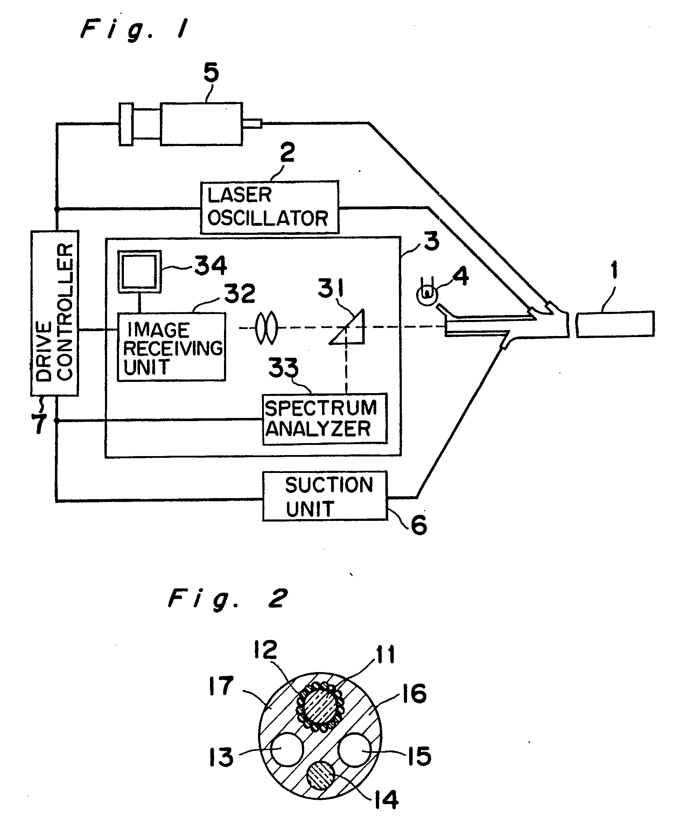

Fig. 1 is a view showing an embodiment of an intravascular laser operating device of the present invention and Fig. 2 shows a sectional view of a catheter. The intravascular laser operating device comprises; an operation catheter (1) accommodating an illumination light transmission path, an image transmission path, a laser light leading path, a flush liquid passing through hole and a suction path (see Fig. 2), a laser oscillating unit (2), an image device (3) for performing such as a formal display of an endoscopic image and analysis display of a fluorescent spectrum and record of an image data, an illumination light source (4) for entering a visible light in the illumination light transmission path, a flush liquid feeding unit (5) for feeding the flush liquid into the flush liquid passing through hole, a suction unit (6) for sucking waste after projecting the laser through the suction path, and a driving controller (7) for setting the time periods of an endoscopic display and analyzing display by the image device (3), the time for laser projection, and the time periods for feeding and sucking the flush liquid and besides for setting the driving turns and driving timings of the endoscopic display, analyzing display, laser oscillating unit (2), illumination light source (4), flush liquid feeding unit (5) and suction unit (6).

With further explanation, the catheter (1) is formed in such a manner that an image transmission path (11), an illumination light transmission path (12) formed in the peripherals of the image transmission path (11), a flush liquid passing through hole (13), a laser light leading path (14) and sucking path (15) are fixed in a flexible medium (16) and the surface thereof is coated by a thin film (17). The outer diameter of said catheter (1) is made extremely thinned diameter of a few milli meters, preferably less than 1.5 mm. The above image transmission path (11) has an objective lens attached to its end portion. In particular, in order to accomplish an image forming with high quality, there is used a material with low dispersion and the high precision of the optical systems of the both edges can be realized.

As the laser oscillating unit (2), since the laser light is largely absorbed in a tissue and is little decreased in flush liquid such as blood or isotonic sodium chloride solution, it is desirable to use a pulse oscillation excimer laser of noble gas halide such as XeCl , KrF and ArF, which can effectively remove a focus. And the control of the output of the laser oscillating unit (2) is performed by adjusting the output power, pulse width and pulse repetition frequency through the driving controller (7). Moreover, the output of the laser light is controlled and a low output laser light (ultraviolet range) is projected to the diseased part and the fluorescent light from the diseased part is led through the image transmission path (11) so that the fluorescent light is spectrum-analyzed, whereby the condition of the diseased part can be diagnosed.

Since the laser oscillating unit in use generates a laser light in the ultraviolet range, the laser light leading path (14) is made of materials capable of transmitting the ultraviolet rays with high energy density and with low loss so that a material with good transmittance such as quartz is used and the end faces thereof are processed with high precision in order to suppress the heat generation at the end faces. The illumination light transmission path (12) is made using visible light transmittable materials such as multi-components group glass or plastic resin with high resiliency, projecting an illumination light from the peripherals of the tip of the image transmission path (11). Moreover, it is possible to accommodate a control wire in the catheter (1) for facilitating to conduct the tip portion of the catheter (1) to the diseased part and it is also possible to provide a balloon for stopping and fixing blood flow on the tip portion of the catheter (1).

The image device (3) comprises; a dividing optical system (31) for dividing the output light of the image transmission path (11), an image receiving unit (32) for receiving one of the divided light by CCD elements, a spectrum analyzing unit (33) for obtaining the spectrum components of the other divided light and of the fluorescent light generated by the diseased part corresponding to the low output laser projection, a monitor television (34) for displaying the processed image signals on the television screen and a VTR (video tape recorder) for recording the image.

The illumination light source (4) comprises an Xe lamp, entering visible light into the illumination light transmission path (12) and projecting the light into a vessel.

The flush liquid feeding unit (5) comprises a pump and is combined with the flush liquid passing through hole (13) through which the flush liquid is fed to the peripherals of the diseased part.

The suction unit (6) comprises a vacuum pump of which the suction mouth is connected to the suction path (15). And the waste after projecting the laser is sucked through the suction path (15).

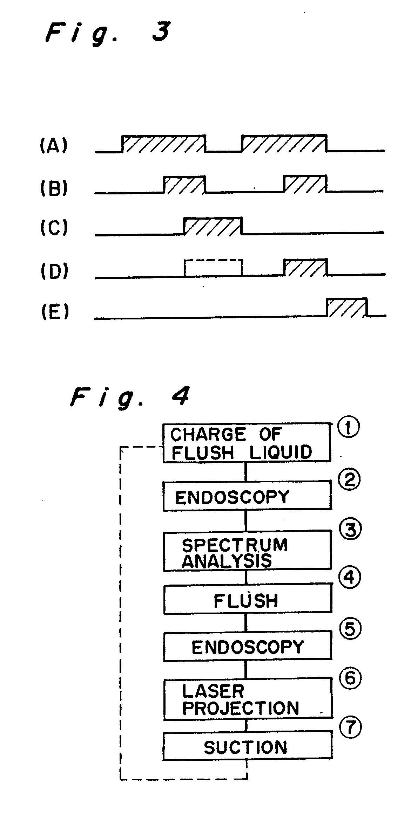

The driving controller (7) accommodating a microcomputer performs the adjustment of the pulse width and the pulse repetition frequency of the laser light and performs the drive, stop and power adjustment of the illumination light source (4), flush liquid feeding unit (5) and suction unit (6) and furthermore sets the time for displaying the diseased part and the time for spectrum analysis of the image device (3). Explaining further in detail with reference to Fig. 3, the driving controller (7) sets the flush liquid feeding time (oblique lines portion A in Fig. 3), endoscopic time, i.e., the time for displaying the image of the diseased part on the image device (3) (oblique lines portion B in Fig. 3), the time for projecting low output laser light (broken lines portion D in Fig. 3), the spectrum analyzing time of fluorescent light obtained through the image transmission path (11) (oblique lines portion C in Fig. 3), the laser light projecting time (oblique lines portion D in Fig. 3) and the suction time (portion E in Fig. 3) and furthermore sets the turns and timings of the flush liquid feeding, endoscopic display, spectrum analysis, laser light projection and suction. The above mentioned spectrum analysis means an analysis of fluorescent light of the diseased part for the laser light of low output and the time required for the spectrum analysis corresponds to the projection time of the low output laser light. In this case, in the observation of the spectrum mentioned above, there is previously given a drug agent (marker such as hematoporphyrin derivative) selectively adhering to the diseased part, and upon observing the fluorescent spectrum peculiar to the marker generated from the corresponding adhering portion, it becomes also possible to facilitate to find the diseased part. Moreover, the endoscopic display and spectrum analyzing display mentioned above may be always displayed on the image device (3).

Next, the operation of the intravascular laser operating device mentioned above is explained with reference to the flow chart of Fig. 4.

The operator conducts the catheter (1) into a predetermined vessel (such as a coronary artery). And the flow is started by the driving controller (7) as follows. That is to say,

(1) The flush liquid is charged into the flush liquid passing through hole (13) and is replaced with the blood in the diseased part so as to make transparent (see Fig. 3A).

(2) The inside of the vessel is displayed on the monitor television (34). The operator examines whether or not a diseased part is present by observing the monitor television (34). In other words, the operator performs an endoscopy (see Fig. 3B).

(3) The laser light of low output is projected to the diseased part in a timing delayed than the endoscopic timing mentioned above and the spectrum analyzing result is displayed on the television (see Figs. 3C, D).

(4) After displaying the spectrum analysis, the flush liquid is once more charged into the flush liquid passing through hole (13) and is replaced with the blood in the diseased portion to be made transparent, and

(5) the diseased portion is displayed on the monitor television (34) and the operator performs the endoscopy (see left side pulse in Figs. 3A and B).

(6) The laser light is projected to the diseased portion to be removed at the same time of the endoscopy (see Fig. 3D). That is, the result of the diseased portion after projection can be immediately confirmed.

(7) After the above laser is projected, the waste produced in the vessel is sucked by driving the suction unit (6) (see Fig. 3E).

In the embodiment as described above, since the endoscopy time, spectrum analyzing time, laser projecting time, flush liquid blowout time and suction time are individually set by the driving controller unit (7) and the operation can be performed according to the flow from (1) to (7) as described with reference to Figs. 3 and 4, therefore, it is possible to reduce the troublesome operations of the operator after the catheter (1) is inserted to the diseased part in the vessel.

Moreover, since the laser projection timing and projection time after the flush liquid is blown out are set, it is possible to dissolve a fear of injuring a healthy vessel and a fear of remaining the diseased part.

Furthermore, since the laser light is an ultraviolet ray and a pulse oscillation is generated, the light is largely absorbed by the tissue and the pulse can be generated with large peak power and the range of the peripherals of the projection area where heat is affectable can be defined, so that a delicate treatment can be performed with good precision. At this time, since the laser light can be projected while watching the monitor television (34), it can be immediately judged whether or not the destruction is completely performed. If not perfect, the laser light is once more projected.

Furthermore, the output of the laser light is controlled and the low output laser light (in the range of ultraviolet ray) is projected to the diseased part and the fluorescent light from the diseased part is led through the image transmission path (11) and the fluorescent light is spectrum-analyzed, whereby the condition of the diseased part can be diagnosed.

Moreover, the present invention is not limited to the above mentioned embodiment and instead of the catheter (1) accommodating the entire part of the illumination light transmission path (12), image transmission path (11), laser light leading path (14), drug liquid leading path and suction path (15), for example, there may be used a thin catheter (1) combined of a plurality of components of the above components.

Also, as to the laser oscillation unit, it is not limited to an excimer laser but there may be used a laser device in which a third harmonic can be obtained by combining a nonlinear crystal with Q switch Nd:YAG laser for example in the range of ultraviolet ray or with Er:YAG laser in the range of infrared ray.

However, in the case Er:YAG laser is used, there is no need to say that, as the laser light leading path, it is necessary to combine fibers having large transmission energy and little transmission loss for the applied laser using such as fluoride glass fiber or chalcogen glass fiber.

Also, it is not always necessary that the excitation light source is used in common as the laser oscillating unit but e.g. an excimer laser excitation dye laser may be used independently of the laser oscillating unit. Other various modifications of the design may be made within the range of the essence of the present invention.

Moreover, it is possible that the illumination light transmission path serves as the above fluorescent light transmission path and there may be provided an optical path switching unit for leading the fluorescent light to the spectrum analyzing device between the illumination light source and the illumination light transmission path. Also, the laser light leading path may serve as the above fluorescent light transmission path and there may be provided an optical path switching unit for leading the fluorescent light to the spectrum analyzing device between said laser oscillating unit and the laser light leading path. The above mentioned excitation light transmission path may serve as the fluorescent light transmission path and there may be provided an optical path switching unit for leading the fluorescent light to the spectrum analyzing device between the excitation light source and the excitation light transmission path mentioned above. The above mentioned illumination light transmission path may serve as the excitation light transmission path and there may be provided a switching unit for switching for the incident light to be entered in the illumination light transmission path between the illumination light from the illumination light source and the excitation light from the excitation light source.

As described above, according to the intravascular laser operating device of the present invention, since the catheter comprises both of the image transmission path and the laser light leading path and the laser light can be generated by properly controlling the drive of the laser unit depending on the endoscopy image and the spectrum analyzing result, therefore, it is not necessary to replace the fibers, so that it is possible to advance the treatment confirming the condition of the diagnosis all the time. Accordingly, there is obtained a specific effect that the diseased part can be quickly treated with safety and sureness.

In addition, according to the intravascular laser operating device of the present invention, since the times of laser projection, drug liquid blowout and suction can be individually set by the drive controller (7) and the turns and timings of the laser projection, drug liquid blowout and suction can be also set, therefore, after the catheter (1) is inserted to the diseased portion in the vessel, the processes from the drug liquid blowout to the suction of the waste can be automatically performed, so that the operation of the operator can be reduced. Moreover, since the laser projection timing and projecting time are set after blowout of the drug liquid, there is obtained a specific effect that the fear of injuring a healthy vessel and the fear of remain of a diseased part can be dissolved.

## Claims

1. An intravascular laser operating device which displays the inside of a blood vessel on an image device based on output light from a catheter inserted into a body and which applies laser light to a diseased part by a laser device so as to perform a treatment, comprising;

   a catheter accommodating the whole or some of components (1) to (6), i.e., (1) an illumination light transmission path for leading illumination light into a blood vessel, (2) an image transmission path for transmitting an image of the inside of the blood vessel, (3) an excitation light transmission path for leading excitation light into the blood vessel, (4) a fluorescent light transmission path for transmitting fluorescent light into the blood vessel, (5) a laser light leading path for leading laser light to a diseased part in the blood vessel, and (6) a drug liquid passage path for blowing out transparent drug liquid around the diseased part,

   an image device for displaying a form of an endoscopic image depending on the output light obtained through said image transmission path,

   a spectrum analyzing device for analyzing the spectrum of the fluorescent light obtained through said fluorescent light transmission path and displaying the analyzed result,

   an excitation light source for generating excitation light and applying into said excitation light transmission path,

   an illumination light source for generating illumination light and applying into said illumination light transmission path,

   a laser oscillating unit for generating laser light and applying into said laser light leading path,

   a drug liquid blowout device for charging transparent drug liquid into said drug liquid passage path, and

   a drive control unit for controlling the system of the entire part.

2. The intravascular laser operating device according to Claim 1, wherein said drive control unit individually sets a laser projection time, drug liquid blowout time, excitation light projection time and suction time and furthermore sets the turns and timings of the laser light projection, drug liquid blowout, excitation light projection and suction.

3. The intravascular laser operating device according to Claim 1, wherein said drive control unit sets the times for performing the endoscopic display and fluorescent analysis by said image device and/or spectrum analyzing device and also sets the turns and timings of the endoscopic display and fluorescent analysis.

4. The intravascular laser operating device according to Claim 1, further comprising a sucking device for sucking waste which is produced in the blood vessel due to the laser projection through said drug liquid passage path.

5. The intravascular laser operating device according to Claim 4, wherein said catheter independently comprises a suction path for sucking the waste produced due to the projection of the laser light.

6. The intravascular laser operating device according to Claim 1, wherein said laser oscillating unit is used also as said excitation light source and one transmission path is used both as said laser light leading path and said excitation light transmission path.

7. The intravascular laser operating device according to Claim 1, wherein said image transmission path serves as said fluorescent light transmission path and said image device displays a form of an endoscopic image based on the output light obtained through the image transmission path and analyzes the spectrum of the fluorescent light obtained through the image transmission path so as to display the analyzed result.

8. The intravascular laser operating device according to Claim 1, wherein said illumination light transmission path serves as said fluorescent light transmission path and there is provided an optical path switching unit for leading the fluorescent light to the spectrum analyzing device between the illumination light source and the illumination light transmission path.

9. The intravascular laser operating device according to Claim 1, wherein said laser light leading path serves as said fluorescent light transmission path and there is provided an optical path switching unit for leading the fluorescent light to the spectrum analyzing device between said laser oscillating unit and said laser light leading path.

10. The intravascular laser operating device according to Claim 1, wherein said excitation light transmission path serves as the fluorescent light transmission path and there is provided an optical path switching unit for leading the fluorescent light to the spectrum analyzing device between said excitation light source and said excitation light transmission path.

11. The intravascular laser operating device according to Claim 1, wherein said illumination light transmission path serves as said excitation light transmission path and there is provided a switching unit for switching for the incident light to the illumination light transmission path whether the illumination light from the illumination light source or the excitation light from the excitation light source is applied.

12. The intravascular laser operating device according to Claim 11, wherein said illumination light source serves as said excitation light source.

*Fig. 1*

*Fig. 2*

# Fig. 3

(A)

(B)

(C)

(D)

(E)

# Fig. 4

| CHARGE OF FLUSH LIQUID | ① |
| ENDOSCOPY | ② |
| SPECTRUM ANALYSIS | ③ |
| FLUSH | ④ |
| ENDOSCOPY | ⑤ |
| LASER PROJECTION | ⑥ |
| SUCTION | ⑦ |

# INTERNATIONAL SEARCH REPORT

International Application No    PCT/JP89/00564

## I. CLASSIFICATION OF SUBJECT MATTER (if several classification symbols apply, indicate all) 6

According to International Patent Classification (IPC) or to both National Classification and IPC

Int. Cl⁴    A61B17/36

## II. FIELDS SEARCHED

### Minimum Documentation Searched 7

| Classification System | Classification Symbols |
| --- | --- |
| IPC | A61B17/36, 10/00, A61N5/06 |

### Documentation Searched other than Minimum Documentation to the Extent that such Documents are Included in the Fields Searched 8

| | |
| --- | --- |
| Jitsuyo Shinan Koho | 1968 – 1989 |
| Kokai Jitsuyo Shinan Koho | 1971 – 1989 |

## III. DOCUMENTS CONSIDERED TO BE RELEVANT 9

| Category * | Citation of Document, 11 with indication, where appropriate, of the relevant passages 12 | Relevant to Claim No. 13 |
| --- | --- | --- |
| X | JP, A, 59-40830 (Hamamatsu TV Co., Ltd.) 6 March 1984 (06. 03. 84) (Family : none) | 1-3, 6-12 |
| Y | JP, A, 59-172621 (Sumitomo Electric Industries, Ltd.) 29 September 1984 (29. 09. 84) (Family : none) | 1-2, 4-5 |
| Y | JP, A, 62-277933 (Olympus Optical Co., Ltd.) 2 December, 1987 (02. 12. 87) (Family : none) | 1-2, 4-5 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
| --- | --- |
| August 7, 1989 (07. 08. 89) | August 28, 1989 (28. 08. 89) |
| International Searching Authority | Signature of Authorized Officer |
| Japanese Patent Office | |

Form PCT/ISA/210 (second sheet) (January 1985)